# FASCICULE DE BREVET EUROPEEN

(11) **EP 0 955 930 B1**
(45) Date de publication et mention de la délivrance du brevet: **25.06.2003**
(21) Numéro de dépôt: 97952991.4
(22) Date de dépôt: 23.12.1997
(51) Int. Cl.: A61B 17/70

(54) **SYSTEME D'OSTEOSYNTHESE DU RACHIS AVEC REGLAGE EN POSITION ET OUTILLAGE DE POSITIONNEMENT**
REGULIERBARE OSTEOSYNTHESEVORRICHTUNG FÜR DIE WIRBELSÄULE UND POSITIONIERUNGSWERKZEUG
ADJUSTABLE OSTEOSYNTHETIC SYSTEM OF THE RACHIS AND POSITIONING TOOL

(30) Priorité: 27.12.1996 FR 9616115
(43) Date de publication de la demande: 17.11.1999
(73) Titulaire: Stryker France S.A., 93290 Tremblay-en-France (FR)
(72) Inventeur: LE COU DIC, Régis, F-33160 Saint Médard en Jalles (FR); CONCHY, Frédéric, F-33000 Bordeaux (FR)
(74) Mandataire: Le Forestier, Eric
(86) Numéro de dépôt international: FR9702398
(87) Numéro de publication internationale: WO98029046

(56) Documents cités:
- WO-A-90/02527
- DE-U- 8 712 943
- DE-U- 9 112 466
- US-A- 5 395 370

## Description

L'invention concerne les systèmes d'ostéosynthèse pour colonne vertébrale et les outillages de positionnement pour de tels systèmes.

On connaît d'après le document FR-2 659 546 un système d'ostéosynthèse pour colonne vertébrale comprenant plusieurs vis pédiculaires destinées à être ancrées dans des vertèbres de la colonne vertébrale et une tige de liaison adaptée à relier rigidement les vis pédiculaires entre elles. Un tel système est utilisé notamment en cas de fracture simple ou multiple d'une ou plusieurs vertèbres de la colonne. La fracture peut avoir différentes configurations et nécessiter des mouvements de correction pour retrouver la morphologie originelle du rachis, notamment en ce qui concerne les courbures de lordose ou de cyphose. Pour cela, chaque vis pédiculaire comprend une bague sphérique enfilée à coulissement sur la tige et reçue dans un évidement de la tête de la vis pédiculaire en étant libre de se mouvoir dans cet évidement. Après ancrage des vis dans les vertèbres, on peut donc placer chaque vis pédiculaire à une position quelconque sur la tige et suivant une orientation quelconque par rapport à la tige. La tête de la vis comporte un verrou permettant d'immobiliser ensemble rigidement la vis, la bague et la tige pour fixer rigidement la vis à la tige dans la position voulue. Ce système permet donc à volonté de faire coulisser les vis pédiculaires les unes par rapport aux autres (mouvement de distraction) et de les orienter relativement (correction angulaire), afin de replacer les vertèbres dans la position voulue pour l'ostéosynthèse avant fixation rigide des vis à la tige. Toutefois, ce système qui rend aisés les mouvements de distraction des vis pédiculaires, ne permet pas de réaliser facilement un réglage précis de l'écartement et de l'orientation angulaire des vis pédiculaires entre elles.

Le document DE-91 12466 présente un outillage de positionnement pour un dispositif d'ostéosynthèse. Cet outillage comporte deux bras adaptés à être rapportés sur deux vis pédiculaires de façon à constituer des prolongements de celles-ci. Il comporte également un organe de liaison de bras s'étendant de l'un à l'autre des bras. Cet organe présente deux tiges filetées coopérant par des liaisons vis-écrous avec les bras. Une manoeuvre de l'organe de liaison permet de faire varier la distance entre les bras et, en coopération avec l'organe de liaison des vis, de faire varier l'inclinaison relative des vis. Toutefois, ce dispositif a pour inconvénient que la manoeuvre de l'organe fileté est relativement longue à effectuer, notamment lorsqu'on souhaite produire une grande variation de distance entre les bras. De plus, cette manoeuvre longue requiert de l'opérateur de nombreuses manipulations sur l'outillage, notamment des rotations répétées de l'organe de liaison de bras. Toutes ces manipulations peuvent générer des sollicitations transmises jusqu'aux vertèbres.

Un but de l'invention est de fournir un outillage permettant de faire varier plus rapidement la position relative des bras en évitant de transmettre des sollicitations indésirables aux vertèbres.

En vue de la réalisation de ce but, on prévoit selon l'invention un outillage de positionnement pour un système d'ostéosynthèse pour colonne vertébrale comportant deux organes d'ancrage allongés adaptés chacun à être ancrés dans une vertèbre de la colonne, et un élément de liaison d'ancrage allongé adapté à relier entre eux les organes d'ancrage, le système étant adapté à avoir une configuration non verrouillée dans laquelle chaque organe d'ancrage est mobile à rotation par rapport à l'élément de liaison d'ancrage autour d'un premier axe perpendiculaire à une direction longitudinale de l'organe d'ancrage et perpendiculaire à une direction longitudinale de l'élément de liaison d'ancrage, et une configuration verrouillée dans laquelle chaque organe d'ancrage est rigidement fixé à l'élément de liaison d'ancrage, l'outillage comportant deux bras adaptés à être fixés de façon amovible aux deux organes d'ancrage respectifs et un élément de liaison de bras allongé adapté à relier entre elles une partie de chaque bras, l'élément de liaison de bras étant adapté à relier les parties de bras en définissant un extremum d'écartement mutuel des parties de bras, l'élément de liaison de bras comportant au moins une crémaillère présentant des dents adaptées à venir en prise avec l'une des parties de bras, la ou chaque crémaillère étant fixée à rotation à l'autre partie de bras.

Ainsi, la crémaillère permet de faire varier avec une grande rapidité la distance entre les deux parties de bras reliées par la crémaillère. Pour cela, il suffit de manoeuvrer la crémaillère pour interrompre sa prise avec l'un des bras, puis de modifier la position relative des bras et de remettre la crémaillère en prise avec ce bras. On évite la manoeuvre prolongée de l'outillage. De la sorte, on évite des manipulations nombreuses et répétées et on réduit le risque que des contraintes de réglage soient transmises aux vertèbres. La crémaillère peut très facilement être mise en prise ou hors de prise avec le bras.

L'invention fournit un outillage permettant de réaliser facilement un réglage précis de l'écartement et de l'orientation angulaire des vis pédiculaires entre elles. De plus, l'élément de liaison d'ancrage permet notamment d'effectuer les mouvements de distraction des organes d'ancrage et de fixer l'écartement entre des premières parties des organes reliées à cet élément de liaison. L'élément de liaison de bras permet de limiter l'écartement entre les parties de bras. Etant donné que l'orientation angulaire relative des organes d'ancrage résulte du choix de l'écartement entre les premières parties des organes d'ancrage et du choix de l'écartement entre les parties de bras, on peut choisir facilement et précisément l'orientation angulaire relative des organes d'ancrage. De même, on peut facilement régler avec précision l'écartement mutuel entre les organes d'ancrage.

Avantageusement, l'élément de liaison de bras est adapté de sorte que, lorsqu'il définit l'extremum, il autorise un déplacement relatif des parties de bras dans un unique sens de déplacement.

Avantageusement, l'élément de liaison de bras est adapté de sorte que, lorsqu'il définit l'extremum, chaque bras est mobile à rotation par rapport à l'élément de liaison de bras autour d'un deuxième axe parallèle au premier axe.

Avantageusement, l'extremum est un maximum.

Avantageusement, la ou chaque crémaillère est agencée de sorte que les dents s'étendent en direction des organes d'ancrage lorsque les parties de bras sont reliées par la crémaillère.

Avantageusement, la ou chaque crémaillère est mobile à coulissement par rapport au bras auquel elle est fixée, suivant le deuxième axe associé.

Avantageusement, la partie de bras adaptée à venir en prise avec la ou chaque crémaillère présente pour la ou chaque crémaillère, une zone adaptée à venir en prise avec la crémaillère et ayant une largeur supérieure à une largeur de la crémaillère.

Avantageusement, le bras portant la ou chaque crémaillère comporte des moyens de rappel de la ou des crémaillères vers une position correspondant à la mise en prise des dents avec l'autre partie de bras.

Avantageusement, l'élément de liaison de bras comporte deux crémaillères rigidement fixées l'une à l'autre, coplanaires, parallèles l'une à l'autre, présentant des dents s'étendant dans une même direction et adaptées à s'étendre de part et d'autre du bras avec lequel elles viennent en prise.

Avantageusement, chaque bras est adapté pour être mobile en rotation par rapport à l'organe d'ancrage associé autour d'un axe longitudinal de l'organe d'ancrage.

Avantageusement, chaque organe d'ancrage comprend des moyens de verrouillage de l'organe d'ancrage en position sur l'élément de liaison d'ancrage, le bras associé présentant une ouverture pour l'actionnement des moyens de verrouillage lorsque le bras est fixé à l'organe d'ancrage.

Avantageusement, chaque bras présente une face externe plane adaptée à s'étendre en regard de l'autre bras lorsque les bras sont reliés par l'élément de liaison de bras, cette face étant adjacente à une extrémité libre du bras opposée à l'organe d'ancrage et inclinée par rapport à un axe longitudinal du bras, de sorte que le bras a une largeur se rétrécissant en direction de cette extrémité libre.

Avantageusement, il comporte un outil à main pour rapprocher ou éloigner mutuellement des parties respectives des organes d'ancrage ou des bras reliées par l'un des éléments de liaison.

On prévoit également selon l'invention un ensemble pour l'ostéosynthèse de la colonne vertébrale comprenant un système d'ostéosynthèse comportant deux organes d'ancrage allongés adaptés chacun à être ancrés dans une vertèbre de la colonne, et un élément de liaison d'ancrage allongé adapté à relier entre eux les organes d'ancrage, le système étant adapté à avoir une configuration non verrouillée dans laquelle chaque organe d'ancrage est mobile à rotation par rapport à l'élément de liaison d'ancrage autour d'un premier axe perpendiculaire à une direction longitudinale de l'organe d'ancrage et perpendiculaire à une direction longitudinale de l'élément de liaison d'ancrage, et une configuration verrouillée dans laquelle chaque organe d'ancrage est rigidement fixé à l'élément de liaison d'ancrage, l'ensemble comportant en outre un outillage selon l'invention.

Avantageusement, l'élément de liaison d'ancrage est adapté de sorte qu'en configuration déverrouillée chaque organe d'ancrage est mobile à coulissement le long de l'élément de liaison d'ancrage.

Avantageusement, l'élément de liaison d'ancrage comprend une tige et chaque bras présente au moins une échancrure pour le passage de la tige, l'échancrure ayant une largeur supérieure à un diamètre de la tige.

D'autres caractéristiques et avantages de l'invention apparaîtront encore dans la description qui va suivre d'un mode préféré de réalisation donné à titre d'exemple non limitatif. Aux dessins annexés :
- la figure 1 est une vue en perspective du système selon l'invention une fois installé, les vertèbres n'étant pas représentées ;

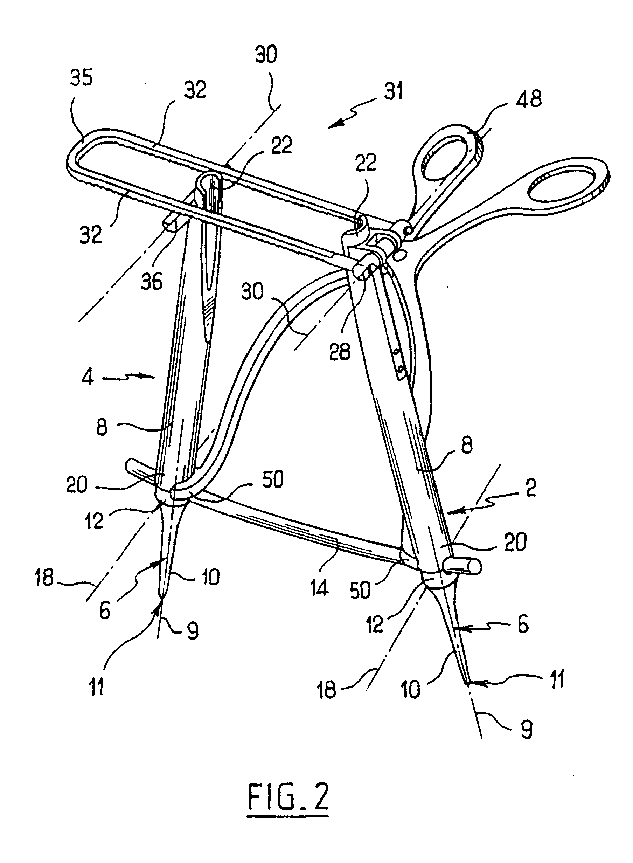

- la figure 2 est une vue analogue montrant l'installation du système de la figure 1, avec la pince distractrice ;
- la figure 3 et la figure 4 sont des vues de dos et de gauche du tube crémaillère des figures 1 et 2 ;
- la figure 5 est une vue partielle en coupe à échelle agrandie du tube crémaillère de la figure 4, montrant l'arbre et le ressort associé en coupe perpendiculaire à l'axe de l'arbre ;
- les figures 6 et 7 sont des vues de dessus du tube crémaillère de la figure 4, montrant différentes positions en translation de l'élément de liaison de bras par rapport au tube associé suivant l'axe de l'arbre ; et
- les figures 8 et 9 sont des vues de dos et de gauche du tube de rappel des figures 1 et 2.

L'équipement d'ostéosynthèse de l'invention comprend deux ensembles tels que celui qui va être décrit en référence aux figures, incluant deux systèmes destinés à être fixés par exemple aux mêmes vertèbres de la colonne, de part et d'autre de l'axe médian de la colonne.

Le système comporte deux organes d'ancrage 6 ou vis pédiculaires ayant une forme rectiligne allongée d'axe longitudinal 9. Chaque vis pédiculaire 6 présente une portion d'extrémité filetée 10 ou pied adaptée à être ancrée dans une vertèbre de la colonne, et une tête 12 opposée longitudinalement à cette portion filetée 10. La vis 6 présente une extrémité libre 11 opposée longitudinalement à la tête 12.

Le système comporte un élément de liaison d'ancrage 14 constitué ici par une tige allongée généralement rectiligne à section circulaire.

Chaque tête de vis 12 comporte, de façon non représentée, deux pattes s'étendant en regard l'une de l'autre, définissant entre elles une cavité. La tête 12 comporte une bague fendue radialement ayant une face externe sphérique et un alésage central cylindrique. La bague est adaptée à être enfilée sur la tige 14 et à être reçue dans la cavité de la tête de vis 12. La vis 6 comprend un anneau de verrouillage, non représenté, coopérant avec les pattes en étant enfilé sur celles-ci pour maintenir l'espacement des pattes lors de leur écartement provoqué par l'action de serrage d'un écrou de verrouillage interne vissé dans la tête de vis et s'appuyant sur la bague sphérique fendue enfilée sur la tige 14. Après verrouillage, la tête de vis est bloquée par l'action combinée de l'écrou interne et de l'anneau de verrouillage. Avant verrouillage, chaque vis pédiculaire 6 est libre de se mouvoir par rapport à la tige 14, notamment en coulissement le long de la tige et en rotation autour de la bague en réalisant une liaison rotule. En particulier, la vis 6 est libre de se mouvoir par rapport à la tige 14 en rotation autour d'un premier axe 18 perpendiculaire à l'axe longitudinal 9 de la vis 6 et perpendiculaire à la direction longitudinale de la tige 14. Ainsi le système peut avoir une configuration non verrouillée dans laquelle les organes d'ancrage 6 sont mobiles par rapport à la tige 14, et une configuration verrouillée dans laquelle les organes d'ancrage sont rigidement fixés à la tige. La configuration non verrouillée permet de choisir la position des organes d'ancrage en fonction de la position souhaitée pour les vertèbres associées, et la configuration verrouillée permet d'immobiliser rigidement les vertèbres dans cette position en vue de l'ostéosynthèse. Les vis pédiculaires 6 et la tige 14 sont généralement connues en soi, par exemple du document FR-2 659 546 précité, et ne seront pas décrites ici plus en détail.

L'outillage comporte deux bras 8 destinés à être associés aux vis respectives et ayant chacun une forme générale allongée rectiligne d'axe 9. Chaque bras 8 comprend un tube de forme générale cylindrique, présentant notamment une face externe cylindrique. Le bras présente une extrémité axiale inférieure 20 et une extrémité axiale supérieure 22. L'extrémité axiale inférieure 20 présente intérieurement deux alésages concentriques d'axe 9 en vue de la fixation de l'extrémité axiale inférieure 20 du bras 8 par emboîtement sur la tête de vis 12 associée. Le bras 8 est ainsi adapté à être fixé à la tête de la vis 12 en s'étendant dans le prolongement de la vis. L'extrémité axiale supérieure 22 du bras 8 s'étend alors à une distance d2 de l'extrémité libre 11 de la vis 6, supérieure au double de la distance d1 séparant l'extrémité axiale inférieure 20 de l'extrémité libre 11 de la vis.

L'extrémité axiale inférieure 20 présente deux échancrures 24 s'étendant dans la paroi du tube et diamétralement opposées de part et d'autre de l'axe 9. Ces échancrures sont adaptées à recevoir la tige 14 lorsque le bras 8 est monté sur la vis 6. Chaque échancrure 24 a une largeur sensiblement supérieure au diamètre de la tige 14 de sorte que, lorsque la tige 14 est reçue dans les échancrures, le bras 8 est mobile à rotation autour de l'axe 9 par rapport à la vis 6 sur une amplitude réduite, par exemple de 10° de chaque côté d'une position relative médiane, et donc de 20° au total. Chaque extrémité axiale supérieure 20 présente une ouverture centrale autorisant le passage d'un outil dans le bras 8 pour l'actionnement de l'écrou de verrouillage de la vis pédiculaire 6 sur la tige 14 tel que précité lorsque le bras 8 est en place sur la tête de vis 12.

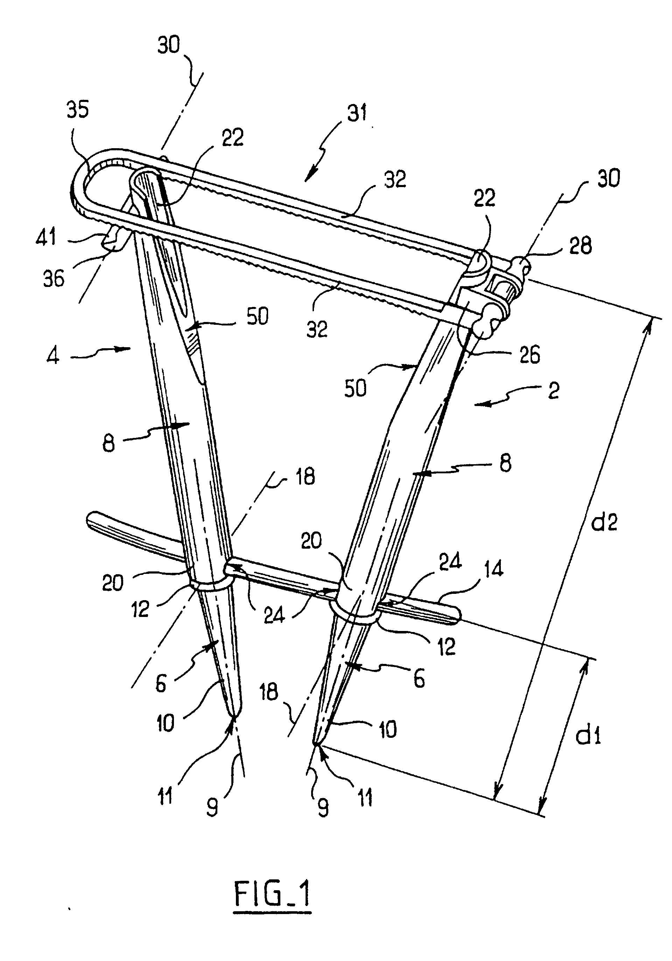

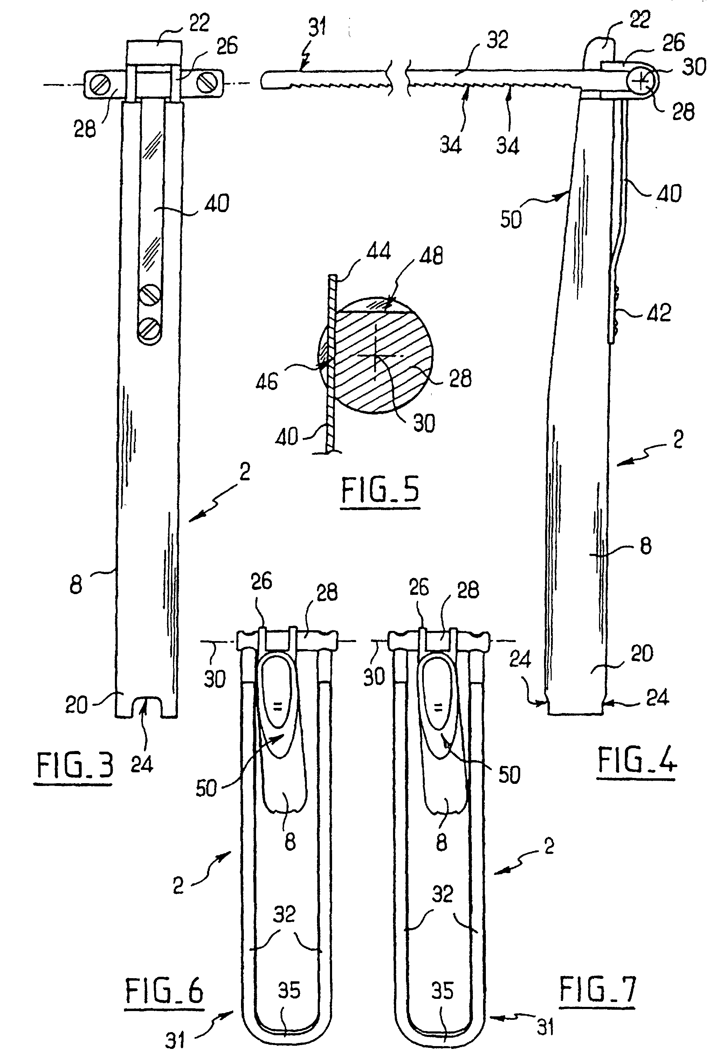

L'un des bras 8, situé à droite sur la figure 1 et représenté sur les figures 3 à 7, porte, au voisinage de son extrémité axiale supérieure 22, une chape 26 traversée par un arbre 28 d'axe 30, mobile à rotation par rapport à la chape autour de l'axe 30 et mobile à translation par rapport à la chape suivant l'axe 30. Nous appellerons cet axe "deuxième axe". Le deuxième axe 30 est perpendiculaire à l'axe longitudinal 9 et à la direction longitudinale de la tige 14. Le deuxième axe 30 est parallèle au premier axe 18. Le système comporte deux crémaillères 32 présentant chacune une série de dents 34. Ces crémaillères sont rectilignes, parallèles entre elles et coplanaires. Les dents 34 des deux crémaillères 32 ont des profils identiques coïncidents. Les deux crémaillères sont fixes l'une par rapport à l'autre, les crémaillères étant définies sur deux branches parallèles entre elles d'une barre repliée en forme de "U". La base 35 du "U" s'étend à une première extrémité des crémaillères, et une deuxième extrémité des crémaillères est fixée rigidement à l'arbre 28. Les crémaillères 32 et l'arbre 28 constituent un élément de liaison de bras 31 du système. Cet élément de liaison est mobile à rotation par rapport à l'un des bras 8 autour du deuxième axe 30, et fixé à demeure à ce bras. Dans la suite, nous appellerons "tube crémaillère" le tube portant les crémaillères.

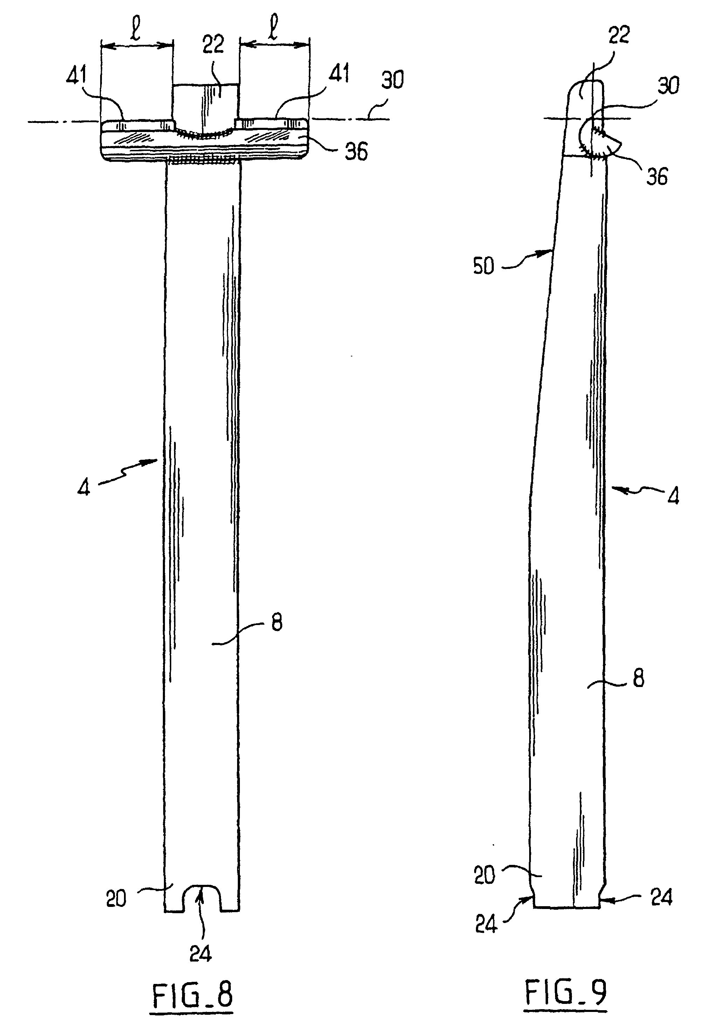

Comme le montrent les figures 7 et 8, la chape 26, l'arbre 28 et les crémaillères 32 sont configurées de sorte que l'arbre 28 est mobile à translation le long du deuxième axe 30 par rapport au bras 8 qui le porte. L'amplitude de cette translation est par exemple de 10 mm. La figure 7 montre une position extrême en translation de l'arbre, l'une des crémaillères étant en appui contre le bras 8. La figure 8 montre une position médiane, les crémaillères 32 étant équidistantes du bras 8.

L'autre bras 8, situé à gauche sur la figure 1 et représenté aux figures 8 et 9, porte une barrette 36 fixée à celui-ci au voisinage de son extrémité axiale supérieure 22. La barrette s'étend suivant une direction parallèle au premier axe 18. La barrette 36 présente sur toute sa longueur un profil en forme de dent, le sommet rectiligne de la dent étant orienté en direction opposée à la vis pédiculaire 6 associée. Ce profil est adapté à coopérer par engagement avec les dents 34 des crémaillères 32 pour être en prise avec l'élément de liaison de bras 31. Nous appellerons "tube de rappel" le tube portant la barrette. Précisément, la barrette 36 présente deux zones 41 s'étendant de part et d'autre du bras 8 associé et destinées à venir en prise avec les deux crémaillères 32 respectives. Chaque zone 41 de la barrette a une largeur 1 supérieure à la largeur de la crémaillère 32 associée en vue de demeurer en prise avec la crémaillère quelle que soit la position de la crémaillère en translation suivant le deuxième axe 30. Il existe deux positions dans lesquelles l'élément de liaison de bras 31 s'étend suivant une direction sensiblement perpendiculaire à l'axe longitudinal 9 du tube crémaillère.

Dans l'une de ces positions, les dents 34 sont orientées en direction de la vis pédiculaire 6 associée et peuvent donc venir en prise avec la barrette 36 du tube de rappel.

Les dents 34 des crémaillères ont un profil dissymétrique en triangle rectangle permettant le déplacement de la barrette 36 le long des crémaillères 32 dans un premier sens lorsque la barrette est en prise avec les crémaillères, et interdisant son déplacement dans un deuxième sens opposé au premier sens. Ainsi, l'élément de liaison de bras 31 permet de définir un extremum d'écartement mutuel entre les deux extrémités axiales supérieures 22 des bras 8. En l'espèce, lorsque la barrette est en prise avec les crémaillères, le profil des dents autorise le rapprochement mutuel des extrémités axiales supérieures 22 des bras et interdit leur éloignement mutuel, et ce à une position quelconque de la barrette sur les crémaillères. L'élément de liaison de bras 31 définit donc ici un maximum d'écartement mutuel.

Le tube crémaillère comporte un ressort 40 constitué par une lamelle plate, allongée, généralement rectiligne, s'étendant parallèlement à l'axe longitudinal 9, ayant une extrémité axiale inférieure 42 fixée à une portion médiane de la face externe cylindrique du bras 8, et une extrémité axiale supérieure 44 en appui contre l'arbre 28 et disposée entre l'arbre 28 et le bras. L'arbre 28 présente deux plats 46, 48 s'étendant sensiblement perpendiculairement l'un à l'autre et parallèlement au deuxième axe 30. Le reste de la face de l'arbre 28 est cylindrique. Le plat 46 est disposé de sorte qu'il réalise un contact surface contre surface avec le ressort 40 lorsque les crémaillères 32 s'étendent perpendiculairement au bras 8 qui le porte en vue de venir en prise avec la barrette 36. Le ressort 40 et le plat 46 constituent des moyens de rappel de l'élément de liaison de bras 31 vers une position d'engagement des crémaillères avec la barrette. De même, le deuxième plat 48 est disposé de sorte que l'élément de liaison de bras 31 est rappelé vers une position de repos où il s'étend sensiblement parallèlement à l'axe longitudinal 9 en appui contre la face cylindrique du bras 8, cette position de repos étant séparée de la position de prise précitée par un angle d'environ 270°.

L'outillage comprend en outre un outil à main tel qu'une pince distractrice 48 présentant deux extrémités d'actionnement 50 adaptées à être appliquées simultanément contre l'extrémité axiale inférieure 20 des bras 8 en vue de les écarter l'une de l'autre.

La barrette 36 et la chape 26 sont disposées de sorte que lorsque les deux bras 8 sont reliés par l'élément de liaison de bras 31, la barrette et la chape s'étendent d'un côté arrière de chaque bras, opposé à l'autre bras. Chaque bras 8 présente une face externe plane 50 s'étendant depuis une zone médiane du bras jusqu'à l'extrémité axiale supérieure 22. Cette face 50 est inclinée par rapport à l'axe longitudinal 9 de sorte que la portion d'extrémité axiale supérieure 22 du bras a une largeur allant en se rétrécissant en direction de cette extrémité 22. Ces faces planes 50 sont disposées de sorte qu'elles s'étendent en regard l'une de l'autre lorsque les deux bras sont reliés par l'élément de liaison de bras 31. Les faces planes 50 sont opposées respectivement à la chape 26 et à la barrette 36 par rapport à l'axe longitudinal 9.

L'élément de liaison de bras 31 et les deux bras 8 constituent un outillage de positionnement du système d'ostéosynthèse.

L'ensemble est utilisé de la façon suivante. On fixe chaque vis pédiculaire 6 à des vertèbres de la colonne, ou à des parties de vertèbre. On met en place les bagues et la tige 14, l'ensemble étant en configuration déverrouillée. On monte chaque bras 8, à savoir le tube crémaillère et le tube de rappel, sur la vis 6 associée. Au moyen de la pince 48, on écarte l'une de l'autre les extrémités axiales inférieures 20 des bras 8, de façon à réaliser un mouvement de distraction des vertèbres associées aux organes d'ancrage 2, 4. La tige 14 sert alors de guide pour le déplacement relatif des organes d'ancrage. Puis, tout en maintenant les extrémités axiales inférieures 20 dans cette position, on fait passer l'élément de liaison de bras 31 de la position de repos en appui contre le bras 8 à la position de mise en prise avec la barrette 36.

On rapproche alors manuellement les deux extrémités axiales supérieures 22 l'une de l'autre comme le permettent les crémaillères 32 en prise avec la barrette 36, pour régler et choisir l'écartement maximum entre les extrémités axiales supérieures des bras. Au cours de ce rapprochement, l'orientation angulaire relative des axes longitudinaux 9 des organes d'ancrage 6 varie. On réalise ainsi un mouvement de correction angulaire de la position des vertèbres.

Lorsque l'écartement souhaité entre les extrémités axiales supérieures 22 est atteint, on relâche ces extrémités 22. Les crémaillères 32 interdisent alors tout éloignement mutuel des extrémités 22 dans cette position (sauf à désengager les crémaillères d'avec la barrette). On peut alors, au besoin, à nouveau modifier l'écartement mutuel entre les extrémités axiales inférieures 20, par exemple au moyen de la pince 48, pour effectuer un nouveau mouvement de distraction et de correction angulaire et ainsi préciser encore la position relative des vertèbres. Au cours de ce mouvement, les extrémités axiales supérieures 22 conservent le même écartement mutuel grâce à l'autostatisme de la liaison entre les crémaillères 32 et la barrette 36. Seule se produit une rotation du tube crémaillère par rapport à l'élément de liaison de bras 31 autour du deuxième axe 30 associé, ainsi qu'une rotation du tube de rappel par rapport à l'élément de liaison de bras 31 autour du deuxième axe 30 associé défini par le sommet de la dent de la barrette 36 en prise avec les crémaillères 32. Ce deuxième axe 30 du tube de rappel est parallèle au deuxième axe 30 du tube crémaillère. On peut ensuite au besoin à nouveau déplacer relativement les extrémités supérieures axiales 22 des bras 8, soit en les rapprochant l'une de l'autre avec la barrette 36 toujours en prise avec les crémaillères 32, soit en les éloignant l'une de l'autre en interrompant provisoirement l'engagement entre la barrette et les crémaillères.

Lorsque les crémaillères 32 sont en prise avec la barrette 36, elles s'étendent de part et d'autre du bras 8. Ainsi, lors du déplacement de la barrette 36 le long des crémaillères pour rapprocher les extrémités 22, les crémaillères constituent un guide pour le mouvement du bras 8 portant la barrette. Les deux faces inclinées 50 permettent de rapprocher les deux extrémités axiales supérieures 22 très près l'une de l'autre, par exemple en les mettant en contact mutuel.

Une fois que la position souhaitée pour les vertèbres a été atteinte, on verrouille les vis pédiculaires 6 rigidement en position sur la tige 14 grâce à l'ouverture précitée ménagée dans les bras à cette fin. Puis on ôte les bras 8 en les séparant des vis pédiculaires 6 respectives.

La liberté de rotation de chaque bras 8 par rapport à la vis 6 associée, et la liberté de translation de l'arbre 28 par rapport à la chape 26 permet au système de s'adapter aux positions souhaitées pour les vertèbres.

L'équipement pourra comporter plusieurs paires d'organes d'ancrage 6 et de bras 8. Il pourra comporter une ou plusieurs tiges 14 pour relier ces organes entre eux.

Les dents 34 de chaque crémaillère 32 pourraient être orientées en sens contraire en vue de définir un minimum d'écartement entre les extrémités axiales supérieures 22 des bras.

La longueur du bras 8 est ici environ le double de la longueur de chaque vis pédiculaire 6. La longueur du bras sera avantageusement supérieure à la longueur de la vis 6 associée.

L'outillage selon l'invention peut être mis en oeuvre avec un dispositif d'ostéosynthèse différent de celui du document de brevet FR-2 659 546.

Par ailleurs, on pourra prévoir plus généralement un outillage de positionnement pour un système d'ostéosynthèse pour colonne vertébrale comportant deux organes d'ancrage adaptés chacun à être ancrés dans une vertèbre de la colonne, et un élément de liaison d'ancrage adapté à relier entre eux les organes d'ancrage, le système étant adapté à avoir une configuration non verrouillée dans laquelle chaque organe d'ancrage est mobile par rapport à l'élément de liaison d'ancrage, et une configuration verrouillée dans laquelle chaque organe d'ancrage est rigidement fixé à l'élément de liaison d'ancrage, outillage comportant deux bras adaptés à être fixés de façon amovible aux deux organes d'ancrage 6 respectifs et un élément de liaison de bras 31 adapté à relier entre elles une partie de chaque bras, dans lequel chaque bras présente une ouverture pour l'actionnement de moyens de verrouillage en position prévus sur chaque organe d'ancrage pendant que le bras est fixé à l'organe d'ancrage.

De préférence, cette ouverture s'étendra suivant l'axe du bras, lequel sera alors creux, de sorte qu'on actionnera les moyens de verrouillage situés près de la base du bras en introduisant un outil dans le bras à partir du sommet du bras.

Par ailleurs, on pourra également prévoir plus généralement un outillage de positionnement pour un système d'ostéosynthèse pour colonne vertébrale comportant deux organes d'ancrage adaptés chacun à être ancrés dans une vertèbre de la colonne, et un élément de liaison d'ancrage adapté à relier entre eux les organes d'ancrage, le système étant adapté à avoir une configuration non verrouillée dans laquelle chaque organe d'ancrage est mobile à coulissement le long de l'élément de liaison d'ancrage, et une configuration verrouillée dans laquelle chaque organe d'ancrage est rigidement fixé à l'élément de liaison d'ancrage, outillage comportant deux bras adaptés à être fixés de façon amovible aux deux organes d'ancrage respectifs et un élément de liaison de bras adapté à relier entre elles une partie de chaque bras. Dès lors, l'outillage comprendra en outre avantageusement un outil à main tel que l'outil 48.

## Revendications

1. Outillage de positionnement pour un système d'ostéosynthèse pour colonne vertébrale comportant deux organes d'ancrage allongés (6) adaptés chacun à être ancrés dans une vertèbre de la colonne, et un élément de liaison d'ancrage allongé (14) adapté à relier entre eux les organes d'ancrage, le système étant adapté à avoir une configuration non verrouillée dans laquelle chaque organe d'ancrage (6) est mobile à rotation par rapport à l'élément de liaison d'ancrage (14) autour d'un premier axe (18) perpendiculaire à une direction longitudinale (9) de l'organe d'ancrage et perpendiculaire à une direction longitudinale de l'élément de liaison d'ancrage (14), et une configuration verrouillée dans laquelle chaque organe d'ancrage (6) est rigidement fixé à l'élément de liaison d'ancrage (14), l'outillage comportant deux bras (8) adaptés à être fixés de façon amovible aux deux organes d'ancrage (6) respectifs et un élément de liaison de bras allongé (31) adapté à relier entre elles une partie (26, 36) de chaque bras, **caractérisé en ce que** l'élément de liaison de bras est adapté à relier les parties de bras en définissant un extremum d'écartement mutuel des parties de bras (26, 36), l'élément de liaison de bras (31) comportant au moins une crémaillère (32) présentant des dents (34) adaptées à venir en prise avec l'une (36) des parties de bras, la ou chaque crémaillère étant fixée à rotation à l'autre partie de bras (26).

2. Outillage selon la revendication 1, **caractérisé en ce que** l'élément de liaison de bras (31) est adapté de sorte que, lorsqu'il définit l'extremum, il autorise un déplacement relatif des parties de bras (26, 36) dans un unique sens de déplacement.

3. Outillage selon l'une quelconque des revendications 1 et 2, **caractérisé en ce que** l'élément de liaison de bras (31) est adapté de sorte que, lorsqu'il définit l'extremum, chaque bras (8) est mobile à rotation par rapport à l'élément de liaison de bras (31) autour d'un deuxième axe (30) parallèle au premier axe (18).

4. Outillage selon l'une quelconque des revendications 1 à 3, **caractérisé en ce que** l'extremum est un maximum.

5. Outillage selon l'une quelconque des revendications 1 à 4, **caractérisé en ce que** la ou chaque crémaillère (32) est agencée de sorte que les dents (34) s'étendent en direction des organes d'ancrage (6) lorsque les parties de bras (26, 36) sont reliées par la crémaillère (32).

6. Outillage selon l'une quelconque des revendications 1 à 5, **caractérisé en ce que** la ou chaque crémaillère (32) est mobile à coulissement par rapport au bras (8) auquel elle est fixée, suivant le deuxième axe associé (30).

7. Outillage selon l'une quelconque des revendications 1 à 6, **caractérisé en ce que** la partie de bras (36) adaptée à venir en prise avec la ou chaque crémaillère (32) présente pour la ou chaque crémaillère, une zone (41) adaptée à venir en prise avec la crémaillère et ayant une largeur (1) supérieure à une largeur de la crémaillère.

8. Outillage selon l'une quelconque des revendications 1 à 7, **caractérisé en ce que** le bras (8) portant la ou chaque crémaillère (32) comporte des moyens de rappel (40) de la ou des crémaillères vers une position correspondant à la mise en prise des dents (34) avec l'autre partie de bras (36).

9. Outillage selon l'une quelconque des revendications 1 à 8, **caractérisé en ce que** l'élément de liaison de bras (31) comporte deux crémaillères (32) rigidement fixées l'une à l'autre, coplanaires, parallèles l'une à l'autre, présentant des dents (34) s'étendant dans une même direction et adaptées à s'étendre de part et d'autre du bras avec lequel elles viennent en prise.

10. Outillage selon l'une quelconque des revendications 1 à 9, **caractérisé en ce que** chaque bras (8) est adapté pour être mobile en rotation par rapport à l'organe d'ancrage associé (6) autour d'un axe longitudinal (9) de l'organe d'ancrage.

11. Outillage selon l'une quelconque des revendications 1 à 10, **caractérisé en ce que** chaque bras (8) présente une ouverture pour l'actionnement de moyens de verrouillage en position prévus sur chaque organe d'ancrage (6), pendant que le bras est fixé à l'organe d'ancrage.

12. Outillage selon l'une quelconque des revendications 1 à 11, **caractérisé en ce que** chaque bras (8) présente une face externe plane (50) adaptée à s'étendre en regard de l'autre bras lorsque les bras sont reliés par l'élément de liaison de bras (31), cette face étant adjacente à une extrémité libre (22) du bras opposée à l'organe d'ancrage (6) et inclinée par rapport à un axe longitudinal (9) du bras, de sorte que le bras a une largeur se rétrécissant en direction de cette extrémité libre (22).

13. Outillage selon l'une quelconque des revendications 1 à 12, **caractérisé en ce qu'**il comprend en outre un outil à main (48) pour rapprocher ou éloigner mutuellement des parties (12) respectives des organes d'ancrage (6) ou des bras (8) reliées par l'un des éléments de liaison.

14. Ensemble d'ostéosynthèse pour colonne vertébrale, **caractérisé en ce qu'**il comprend un système d'ostéosynthèse comportant deux organes d'ancrage allongés (6) adaptés chacun à être ancrés dans une vertèbre de la colonne, et un élément de liaison d'ancrage allongé (14) adapté à relier entre eux les organes d'ancrage, le système étant adapté à avoir une configuration non verrouillée dans laquelle chaque organe d'ancrage (6) est mobile à rotation par rapport à l'élément de liaison d'ancrage (14) autour d'un premier axe (18) perpendiculaire à une direction longitudinale (9) de l'organe d'ancrage et perpendiculaire à une direction longitudinale de l'élément de liaison d'ancrage (14), et une configuration verrouillée dans laquelle chaque organe d'ancrage (6) est rigidement fixé à l'élément de liaison d'ancrage (14), et un outillage selon l'une quelconque des revendications 1 à 13.

15. Ensemble selon la revendication 14, **caractérisé en ce que** l'élément de liaison d'ancrage (14) est adapté de sorte qu'en configuration déverrouillée chaque organe d'ancrage (6) est mobile à coulissement le long de l'élément de liaison d'ancrage (14).

16. Ensemble selon l'une quelconque des revendications 14 ou 15, **caractérisé en ce que** l'élément de liaison d'ancrage (14) comprend une tige et chaque bras (8) présente au moins une échancrure (24) pour le passage de la tige, l'échancrure ayant une largeur supérieure à un diamètre de la tige.

## Patentansprüche

1. Positionierwerkzeug für ein Osteosynthesesystem für eine Wirbelsäule, das zwei verlängerte Ankerorgane (6) umfaßt, die jeweils ausgestaltet sind, in einem Wirbel der Wirbelsäule verankert zu werden, und ein verlängertes Ankerverbindungselement (14), das ausgestaltet ist, die Ankerorgane miteinander zu verbinden, wobei das System so ausgestaltet ist, daß es eine nicht-sperrende Konfiguration, bei der jedes Ankerorgan (6) bezüglich dem Ankerverbindungselement (14) um eine erste Achse (18) drehbeweglich ist, die senkrecht zu einer Längsrichtung (9) des Ankerorgans und senkrecht zu einer Längsrichtung des Ankerverbindungselementes (14) ist, und eine sperrende Konfiguration hat, bei der jedes Ankerorgan (6) starr mit dem Ankerverbindungselement (14) verbunden ist, wobei das Werkzeug zwei Arme (8), die so ausgestaltet sind, daß sie lösbar an den beiden jeweiligen Ankerorganen (6) befestigt sind, und ein verlängertes Armverbindungselement (31) umfaßt, das so ausgestaltet ist, daß es einen Abschnitt (26, 36) jedes Arms miteinander verbindet, **dadurch gekennzeichnet, daß** das Armverbindungselement so ausgestaltet ist, daß es die Armabschnitte verbindet, indem ein Extremum des gegenseitigen Abstands der Armabschnitte (26, 36) definiert wird, wobei das Armverbindungselement (31) wenigstens eine Zahnstange (32) umfaßt, die Zähne (34) aufweist, die so ausgestaltet sind, daß sie in Eingriff mit einem (36) der Armabschnitte gelangen, wobei die oder jede Zahnstange drehfest am anderen Armabschnitt (26) befestigt ist.

2. Werkzeug nach Anspruch 1, **dadurch gekennzeichnet, daß** das Armverbindungselement (31) derart ausgestaltet ist, daß es, wenn es das Extremum definiert, eine Relativverschiebung der Armabschnitte (26, 36) in eine einzige Verschiebungsrichtung zuläßt.

3. Werkzeug nach einem der Ansprüche 1 und 2, **dadurch gekennzeichnet, daß** das Armverbindungselement (31) derart ausgestaltet ist, daß, wenn es das Extremum definiert, jeder Arm (8) bezüglich dem Armverbindungselement (31) um eine zweite Achse (30) drehbeweglich ist, die parallel zur ersten Achse (18) ist.

4. Werkzeug nach einem der Ansprüche 1 bis 3, **dadurch gekennzeichnet, daß** das Extremum ein Maximum ist.

5. Werkzeug nach einem der Ansprüche 1 bis 4, **dadurch gekennzeichnet, daß** die oder jede Zahnstange (32) derart aufgebaut ist, daß die Zähne (34) sich in Richtung der Ankerorgane (6) erstrecken, wenn die Armabschnitte (26, 36) über die Zahnstange (32) verbunden sind.

6. Werkzeug nach einem der Ansprüche 1 bis 5, **dadurch gekennzeichnet, daß** die oder jede Zahnstange (32) bezüglich dem Arm (8) verschiebbar ist, an dem sie befestigt ist, der zugehörigen zweiten Achse (30) folgend.

7. Werkzeug nach einem der Ansprüche 1 bis 6, **dadurch gekennzeichnet, daß** der Armabschnitt (36), der so ausgestaltet ist, daß er in Eingriff mit der oder jeder Zahnstange (32) gelangt, für die oder jede Zahnstange eine Zone (41) aufweist, die so ausgestaltet ist, daß sie in Eingriff mit der Zahnstange gelangt, und eine Breite (1) hat, die größer als eine Breite der Zahnstange ist.

8. Werkzeug nach einem der Ansprüche 1 bis 7, **dadurch gekennzeichnet, daß** der Arm (8), der die oder jede Zahnstange (32) trägt, Mittel (40) zum Rückstellen der Zahnstange(n) in Richtung einer Position umfaßt, die dem Eingriff der Zähne (34) mit dem anderen Armabschnitt (36) entspricht.

9. Werkzeug nach einem der Ansprüche 1 bis 8, **dadurch gekennzeichnet, daß** das Armverbindungselement (31) zwei Zahnstangen (32) umfaßt, die starr aneinander befestigt sind, komplanar sind, parallel zueinander sind, Zähne (34) aufweisen, die sich in die gleiche Richtung erstrecken, und so ausgestaltet sind, daß sie sich beidseits des Arms zu erstrecken, mit dem sie in Eingriff gelangt sind.

10. Werkzeug nach einem der Ansprüche 1 bis 9, **dadurch gekennzeichnet, daß** jeder Arm (8) so ausgestaltet ist, daß er bezüglich dem zugehörigen Ankerorgan (6) um eine Längsachse (9) des Ankerorgans drehbeweglich ist.

11. Werkzeug nach einem der Ansprüche 1 bis 10, **dadurch gekennzeichnet, daß** jeder Arm (8) eine Öffnung für die Betätigung eines Sperrmittels an vorgesehener Position an jedem Ankerorgan (6) aufweist, während der Arm am Ankerorgan befestigt ist.

12. Werkzeug nach einem der Ansprüche 1 bis 11, **dadurch gekennzeichnet, daß** jeder Arm (8) eine ebene Außenfläche (50) aufweist, die so ausgestaltet ist, daß sie dem anderen Arm gegenüberliegend verläuft, wenn die Arme über das Armverbindungselement (31) verbunden sind, wobei diese Fläche in der Nähe eines freien äußeren Endes (22) des Armes liegt, dem Ankerorgan (6) gegenüberliegt, und bezüglich einer Längsachse (9) des Arms geneigt ist, derart, daß der Arm eine Breite hat, die sich in Richtung dieses freien äußeren Endes (22) verjüngt.

13. Werkzeug nach einem der Ansprüche 1 bis 12, **dadurch gekennzeichnet, daß** es außerdem ein Handwerkzeug (48) zum einander Annähern oder Entfernen der jeweiligen Abschnitte (12) der Ankerorgane (6) oder der Arme (8) umfaßt, die von einem der Verbindungselemente verbunden sind.

14. Osteosyntheseausrüstung für eine Wirbelsäule, **dadurch gekennzeichnet, daß** sie ein Osteosynthesesystem, daß zwei verlängerte Ankerorgane (6), die jeweils so ausgestaltet sind, daß sie in einem Wirbel der Wirbelsäule verankert werden, und ein verlängertes Ankerverbindungselement (14) aufweist; das so ausgestaltet ist, daß es die Ankerorgane miteinander verbindet, wobei das System so ausgestaltet ist, daß es eine nicht-sperrende Konfiguration, bei der jedes Ankerorgan (6) bezüglich dem Ankerverbindungselement (14) um eine erste Achse (18) drehbeweglich ist, die senkrecht zu einer Längsachse (9) des Ankerorgans und senkrecht zu einer. Längsrichtung des Ankerverbindurigselementes (14) ist, und eine sperrende Konfiguration hat, bei der jedes Ankerorgan (6) starr am Ankerverbindungselement (14) befestigt ist, und ein Werkzeug nach einem der Ansprüche 1 bis 13 umfaßt.

15. Ausrüstung nach Anspruch 14, **dadurch gekennzeichnet, daß** das Ankerverbindungselement (14) derart ausgestaltet ist, daß in der nicht-sperrenden Konfiguratiön jedes Ankerorgan (6) entlang des Ankerverbindungselementes (14) verschiebbar ist.

16. Ausrüstung nach einem der Ansprüche 14 oder 15, **dadurch gekennzeichnet, daß** das Ankerverbindungselement (14) eine Stange umfaßt, und jeder Arm (8) wenigstens einen Ausschnitt (24) aufweist für den Durchgang der Stange aufweist, wobei der Ausschnitt eine Breite hat, die kleiner als ein Durchmesser der Stange ist.

## Claims

1. Positioning equipment for an osteosynthesis system for a spinal column comprising two elongate anchoring members (6) each designed to be anchored in a vertebra of the column, and an elongate anchoring linking element (14) designed to connect the anchoring members together, the system being designed to have an unlocked configuration in which each anchoring member (6) can turn with respect to the anchoring linking element (14) about a first axis (18) perpendicular to a longitudinal direction (9) of the anchoring member and perpendicular to a longitudinal direction of the anchoring linking element (14), and a locked configuration in which each anchoring member (6) is rigidly fixed to the anchoring linking element (14), the equipment comprising two arms (8) designed to be fixed removably to the two respective anchoring members (6) and an elongate arm linking element (31) designed to connect part (26, 36) of each arm together, **characterized in that** the arm linking element is designed to connect the arm parts, defining an extremum for the mutual separation of the arm parts (26, 36), the arm linking element (31) comprising at least one rack (32) which has teeth (34) designed to mesh with one (36) of the arm parts, the or each rack being fixed, with the ability to rotate, to the other arm part (26).

2. Equipment according to Claim 1, **characterized in that** the arm linking element (31) is designed such that when it defines the extremum, it allows a relative displacement of the arm parts (26, 36) in just one direction of travel.

3. Equipment according to either one of Claims 1 and 2, **characterized in that** the arm linking element (31) is designed such that when it defines the extremum, each arm (8) can rotate with respect to the arm linking element (31) about a second axis (30) parallel to the first axis (18).

4. Equipment according to any one of Claims 1 to 3, **characterized in that** the extremum is a maximum.

5. Equipment according to any one of Claims 1 to 4, **characterized in that** the or each rack (32) is arranged such that the teeth (34) extend towards the anchoring members (6) when the arm parts (26, 36) are connected by the rack (32).

6. Equipment according to any one of Claims 1 to 5, **characterized in that** the or each rack (32) is able to slide with respect to the arm (8) to which it is fixed, in the direction of the associated second axis (30).

7. Equipment according to any one of Claims 1 to 6, **characterized in that** the arm part (36) designed to engage with the or each rack (32) has, for the or each rack, a zone (41) that is designed to engage with the rack and has a width (1) which exceeds the width of the rack.

8. Equipment according to any one of Claims 1 to 7, **characterized in that** the arm (8) bearing the or each rack (32) comprises means (40) for returning the rack or racks to a position that corresponds to the meshing of the teeth (34) with the other arm part (36).

9. Equipment according to any one of Claims 1 to 8, **characterized in that** the arm linking element (31) comprises two racks (32) rigidly fixed to one another, coplanar, parallel to one another, having teeth (34) extending in the one same direction, and designed to extend one on each side of the arm with which they engage.

10. Equipment according to any one of Claims 1 to 9, **characterized in that** each arm (8) is designed to be able to rotate with respect to the associated anchoring member (6) about a longitudinal axis (9) of the anchoring member.

11. Equipment according to any one of Claims 1 to 10, **characterized in that** each arm (8) has an aperture for actuating the position-locking means provided on each anchoring member (6) while the arm is fixed to the anchoring member.

12. Equipment according to any one of Claims 1 to 11, **characterized in that** each arm (8) has a planar external face (50) designed to lie facing the other arm when the arms are connected by the arm linking element (31), this face being adjacent to a free end (22) of the arm which is at the opposite end from the anchoring member (6) and being inclined with respect to a longitudinal axis (9) of the arm so that the arm has a width which tapers towards this free end (22).

13. Equipment according to any one of Claims 1 to 12, **characterized in that** it further comprises a hand tool (48) for moving the respective parts (12) of the anchoring members (6) or of the arms (8) connected by one of the linking elements closer together or further apart.

14. Osteosynthesis set for a spinal column, **characterized in that** it comprises an osteosynthesis system comprising two elongate anchoring members (6) each designed to be anchored in a vertebra of the column, and an elongate anchoring linking element (14) designed to connect the anchoring members together, the system being designed to have an unlocked configuration in which each anchoring member (6) can turn with respect to the anchoring linking element (14) about a first axis (18) perpendicular to a longitudinal direction (9) of the anchoring member and perpendicular to a longitudinal direction of the anchoring linking element (14), and a locked configuration in which each anchoring member (6) is rigidly fixed to the anchoring linking element (14), and equipment according to any one of Claims 1 to 13.

15. Set according to Claim 14, **characterized in that** the anchoring linking element (14) is designed such that in the unlocked configuration each anchoring member (6) can slide along the anchoring linking element (14).

16. Set according to either one of Claims 14 or 15, **characterized in that** the anchoring linking element (14) comprises a rod and each arm (8) has at least one cutout (24) for the passage of the rod, the width of the cutout exceeding a diameter of the rod.
